# EUROPEAN PATENT SPECIFICATION

(11) **EP 2 344 188 B1**
(45) Date of publication and mention of the grant of the patent: **15.02.2017**
(21) Application number: 09785720.5
(22) Date of filing: 02.10.2009
(51) Int. Cl.: A61K 39/07

(54) **ANTHRAX VACCINE FORMULATION AND USES THEREOF**
ANTHRAX-IMPFSTOFF-FORMULIERUNG UND IHRE VERWENDUNGEN
FORMULATION DE VACCIN CONTRE LE CHARBON ET SES UTILISATIONS

(30) Priority: 02.10.2008 US 194967 P
(43) Date of publication of application: 20.07.2011
(73) Proprietor: Pharmathene Inc., Annapolis MD 21401 (US)
(72) Inventor: WATKINSON, Allan, Cleveland TS14 6GG (GB); WOODHOUSE, David, Cleveland TS15 9NX (GB); WILSON, Robert, Cumbernauld G68 9BH (GB)
(74) Representative: Williams, Gareth Owen
(86) International application number: PCT/GB2009/051293
(87) International publication number: WO 2010/038076

(56) References cited:
- WO-A1-2009/093072
- JENDREK S ET AL: "Evaluation of the compatibility of a second generation recombinant anthrax vaccine with aluminum-containing adjuvants" VACCINE, BUTTERWORTH SCIENTIFIC. GUILDFORD, GB, vol. 21, no. 21-22, 20 June 2003 (2003-06-20), pages 3011-3018, XP004429702 ISSN: 0264-410X
- RINELLA J V ET AL: "Treatment of aluminium hydroxide adjuvant to optimize the adsorption of basic proteins" VACCINE, BUTTERWORTH SCIENTIFIC. GUILDFORD, GB, vol. 14, no. 4, 1 March 1996 (1996-03-01), pages 298-300, XP004057327 ISSN: 0264-410X
- RINELLA JOSEPH V JR ET AL: "Effect of Anions on Model Aluminum-Adjuvant-Containing Vaccines" JOURNAL OF COLLOID AND INTERFACE SCIENCE, vol. 172, no. 1, 1 June 1995 (1995-06-01), pages 121-130, XP002558376 ISSN: 0724-8741
- RINELLA JOSEPH V JR ET AL: "Desorption of ovalbumin from aluminum hydroxide adjuvant by competing anions" PHARMACEUTICAL RESEARCH, KLUWER ACADEMIC PUBLISHERS, NEW YORK, NY, US, vol. 10, no. 10, suppl, 18 November 1993 (1993-11-18), page S170, XP009126503 ISSN: 0724-8741
- IYER SEEMA ET AL: "Effect of the degree of phosphate substitution in aluminum hydroxide adjuvant on the adsorption of phosphorylated proteins" PHARMACEUTICAL DEVELOPMENT AND TECHNOLOGY, NEW YORK, NY, US, vol. 8, no. 1, 1 January 2003 (2003-01-01), pages 81-86, XP009126496 ISSN: 1083-7450
- A. WATKINSON ET AL: 'Increasing the Potency of an Alhydrogel-Formulated Anthrax Vaccine by Minimizing Antigen-Adjuvant Interactions' CLINICAL AND VACCINE IMMUNOLOGY vol. 20, no. 11, 28 August 2013, pages 1659 - 1668, XP055213166 DOI: 10.1128/CVI.00320-13 ISSN: 1556-6811
- H. S. HEINE ET AL: 'Determination of Antibiotic Efficacy against Bacillus anthracis in a Mouse Aerosol Challenge Model' ANTIMICROBIAL AGENTS AND CHEMOTHERAPY vol. 51, no. 4, 01 April 2007, pages 1373 - 1379, XP055213167 DOI: 10.1128/AAC.01050-06 ISSN: 0066-4804

## Description

This application claims priority of U.S. Provisional Application 61/194,967, filed 2 October 2008.

### GOVERNMENT RIGHTS NOTICE

The present invention was produced under government contract N01-AI-30052 with the U.S. National Institutes of Allergy and Infectious Diseases (NIAID), which was subsequently transferred to U.S. Biomedical Advanced Research and Development Authority (BARDA) (and renumbered HSSO100200900103C) so that the U.S. Government may have rights in any patent that issues.

### FIELD OF THE INVENTION

The present invention relates to the field of vaccine formulations, such as that containing anthrax protective antigen, and uses of such vaccine, whereby efficacy of such vaccine is both maintained and enhanced.

### Background of the Invention

Vaccines are commonly formulated using an adjuvant. A common adjuvant-type is the aluminum based colloids, usually referred to as alum. More specifically these are usually aluminum hydroxide (aluminum oxyhydroxide) (also called alhydrogel) and aluminum phosphate (also called adju-phos). For example, vaccines useful against organisms such as anthrax (*Bacillus anthracis*) are commonly formulated with alhydrogel, which binds the anthrax antigen used in such vaccines (so called subunit vaccines). One aim of such product formulations was to maximize the binding of rPA to the alum. Since phosphate ions were known to desorb antigen from the alhydrogel colloid, the phosphate concentration in such formulations has been kept deliberately low (at 0.25mM). At this concentration the phosphate buffer does not interfere with rPA binding to the alhydrogel colloid. For example, with this formulation we have found that recombinant protective antigen (rPA) binding was >98%.

Batches of this drug product formulation have been used in the Phase I and Phase II clinical studies, which demonstrated safety and immunogenicity in man.

With this original formulation the pH was found to be 5.9 due to the insufficient buffering capacity of the low phosphate buffer. In order to increase the stability of the rPA drug product and provide a more physiological pH, it was decided that increased control of the formulation was required. The pH of the drug product formulation had to be increased to pH 7 so that an improvement in the buffering capacity of the formulation would be required. For control at pH 7.0, a possible physiological buffer is phosphate, which has a pKa at 7.2 but the disadvantage of using phosphate in the formulation was the inhibitory effect upon rPA-alum binding.

Based on pKa, the possible alternative would be histidine (pKa 6.04), however when stored as a liquid, it has the propensity to oxidize and produce a brown coloration. Moreover, introduction of an alternative buffer such as histidine would mean a radical change in the formulation. A new approach was therefore needed.

We subsequently discovered that we could increase phosphate concentration to a level that both controlled pH and afforded minimal effect on rPA:alhydrogel binding. Consequently, the phosphate concentration was increased to approximately 4 mM; a concentration that was capable of maintaining the Drug Product at a pH of approximately 7.0, and did not markedly affect the amount of unbound rPA, which remained below the level of detection of the assay (<2%).

This led to the surprising result that such a phosphate concentration also greatly increases the bioactivity of the vaccine formulation.

Jendrek et al (Vaccine, vol. 21, no. 21-22, Page 3011 - 3018), describe binding in vitro studies of rPA with alhydrogel. Rinella et al (Vaccine, vol. 14, no. 4, page 298 - 300) describe treatment of aluminium hydroxide adjuvant with phosphate to alter its charge and ability to adsorb proteins. Rinella et al (Journal Of Colloid And Interface Science, vol. 172, no. 1, page 121 - 130) describe the effect of adding phosphate, sulfate or nitrate ions to model aluminium adjuvant containing vaccines. Rinella et al (Pharmaceutical Research, vol. 10, no. 10, suppl, page S170) describe the desorption of ovalbumin from aluminium hydroxide adjuvant by competing anions. lyer et al (Pharmaceutical Development And Technology, vol. 8, no. 1, page 81 - 86) describe the effect of varying phosphate concentration on adsorption of phosphorylated proteins onto aluminium hydroxide adjuvant. Heine et al (Antimocrobial Agents and Chemotherapy, vol. 51, no. 4, page 1373 - 1379) describe an anthrax spore aerosol infection mouse model for determining efficacy of antibiotics.

### BRIEF SUMMARY OF THE INVENTION

In one aspect, the present invention relates to a composition for use as an anthrax vaccine, comprising a therapeutically effective amount of an anthrax protective antigen (PA) bound to adjuvant, wherein said adjuvant is alhydrogel (aluminium hydroxide) and containing a phosphate salt at a concentration in the range of 2 to 5 mM. Other features of the invention are set forth in the dependent claims.

In one embodiment, the vaccine composition is in the form of a lyophilized powder. In another embodiment, the vaccine composition may also comprise phosphate buffered saline (PBS).

In other examples of the vaccine composition or formulation of the invention, the pH of the vaccine composition is in the range of 7.0 to 7.2, preferably about 7.1.

In other examples, the anthrax vaccine formulation of the invention contains alhydrogel (alum) at about 0.15 to 0.35%, preferably at about 0.20 to 0.30%, more preferably at about 0.24 to 0.28%, and most preferably wherein the alhydrogel (alum) is present at about 0.26%.

In a preferred embodiment, the vaccine of the invention is an anthrax vaccine comprising rPA present at about 200 µg/ml, wherein phosphate is present at 4 mM, wherein alhydrogel is present at 0.26% by weight and wherein the pH of said vaccine is 7.1.

The present disclosure further relates to a method of protecting against a bacterial infection in a mammal, comprising administering to a mammal at risk of such infection a therapeutically-effective amount of the vaccine composition of the invention. In further embodiments thereof, the bacterial infection is an anthrax (*Bacillus anthracis*) infection and/or the mammal is a human being.

The present disclosure also relates to a method of treating a bacterial infection in a mammal, comprising administering to a mammal afflicted with such infection a therapeutically-effective amount of the vaccine composition of the invention. In further embodiments thereof, the bacterial infection is an anthrax (*Bacillus anthracis*) infection and/or the mammal is a human being.

### BRIEF DESCRIPTION OF THE DRAWINGS

Figure 1 shows a flowchart for manufacture of a recombinant Protective Antigen (rPA) drug product.
Figure 2 shows the results of an ED50 comparison of high and low phosphate drug products.
Figure 3 shows the effect of phosphate on the zeta potential of alhydrogel diluent and drug product.
Figure 4 shows the effects of phosphate on unbound rPA.
Figure 5 shows the effect of phosphate on adsorptive capacity and adsorptive coefficient of rPA binding to alhydrogel as determined by Langmuir analysis.
Figure 6 shows the effect of phosphate on secondary structure of rPA in a formulated drug product.
Figure 7 shows the effect of phosphate on tertiary structure of rPA in formulated Drug Product.
Figure 8 shows the effect of phosphate on rPA melting as determined by DSC.
Figure 9 shows the effect of phosphate on rPA melting as determined by thermal denaturation/intrinsic fluorescence.
Figure 10 shows epitope recognition of Drug Product formulated at different phosphate concentrations.

### DETAILED DESCRIPTION OF THE INVENTION

The present invention provides an anthrax subunit vaccine formulation containing comprising a therapeutically effective amount of anthrax protective antigen (rPA) in a pharmaceutically acceptable carrier and containing a phosphate salt at a concentration of between 2 and 5 mM, preferably between 3 and 5 mM, more preferably about 4 mM, using alhydrogel as adjuvant. The present disclosure also provides methods for treating or preventing bacterial infections, especially infections of anthrax in a human being, by administering a therapeutically effective amount of a vaccine of the invention.

As used herein the term "effective amount" or "therapeutically effective amount" means a dosage sufficient to treat, inhibit, or alleviate one or more symptoms of a bacterial infection, especially anthrax infection. The precise dosage may vary with such factors as the age, immune system status, general health, and environmental circumstances of the patient, the nature and extent of the infection (or anticipated infection) and the availability of subsequent treatment/vaccination.

Generally, vaccines are prepared as injectables, in the form of aqueous solutions or suspensions. Vaccines in an oil base are also well known such as for inhaling. Solid forms which are dissolved or suspended prior to use may also be formulated. Pharmaceutically acceptable carriers, diluents and excipients are generally added that are compatible with the active ingredients and acceptable for pharmaceutical use.

The pharmaceutical compositions useful herein also contain a pharmaceutically acceptable carrier, including any suitable diluent or excipient, which includes any pharmaceutical agent that does not itself induce the production of antibodies harmful to the individual receiving the composition, and which may be administered without undue toxicity. Pharmaceutically acceptable carriers include, but are not limited to, liquids such as water, saline, glycerol and ethanol, and the like, including carriers useful in forming sprays for nasal and other respiratory tract delivery or for delivery to the ophthalmic system. A thorough discussion of pharmaceutically acceptable carriers, diluents, and other excipients is presented in REMINGTON'S PHARMACEUTICAL SCIENCES (Mack Pub. Co., N.J. current edition).

Vaccine compositions may further incorporate additional substances to stabilize pH, or to function as adjuvants, wetting agents, or emulsifying agents, which can serve to improve the effectiveness of the vaccine.

Vaccines are generally formulated for parenteral administration and are injected either subcutaneously or intramuscularly. Such vaccines can also be formulated as suppositories or for oral administration, using methods known in the art, or for administration through nasal or respiratory routes.

The amount of vaccine sufficient to confer immunity to pathogenic bacteria, viruses, or other microbes is determined by methods well known to those skilled in the art in view of the guidance provided herein. This quantity will be determined based upon the characteristics of the vaccine recipient and the level of immunity required (as already noted above). Where vaccines are administered by subcutaneous or intramuscular injection, a range of 0.5 to 500 µg purified protein may be given. As useful in the present invention, such dosages are commonly sufficient to provide about 1 µg, possibly 10 µg, even 50 µg, and as much as 100 µg, up to 500 µg of immunogenic protein, or immunogenic polypeptide, or immunogenically active fragments thereof. In addition, more than one such active material may be present in the vaccine. Thus, more than one antigenic structure may be used in formulating the vaccine, or vaccine composition to use in the methods disclosed herein. This may include two or more individually immunogenic proteins or polypeptides, proteins or polypeptides showing immunogenic activity only when in combination, either quantitatively equal in their respective concentrations or formulated to be present in some ratio, either definite or indefinite.

A vaccine composition for use in the processes disclosed herein may include one or more immunogenic proteins, one or more immunogenic polypeptides, and/or one or more immunogenically active immunogens comprising antigenic fragments of said immunogenic proteins and polypeptides, the latter fragments being present in any proportions selected by the use of the present invention. The exact components, and their respective quantities, making up the vaccines, and vaccine compositions, useful in the methods of the present invention are determined, *inter alia,* by the nature of the disease to be treated or prevented, the severity of such condition where it already exists, the age, sex, and general health of the recipient, as well the personal and professional experience and inclinations of the researcher and/or clinician utilizing these methods.

For a vaccine of the present invention, such as a sub-unit vaccine for use against anthrax, specific non-limiting examples of this vaccine composition are those wherein said rPA is present at about 10 to 300 µg/ml, preferably 50 to 300 µg/ml, or wherein said rPA is present at about 100 to 300 µg/ml, more preferably about 150 to 250 µg/ml, and most preferably wherein said rPA is present at about 200 µg/ml.

In other examples, the dose of antigen, preferably rPA, to be administered is at least about 5 µg, or at least about 10 µg, or at least about 25 µg, or at least about 50 µg, or at least about 75 µg, or at least about 100 µg, or at least about 150 µg, or at least about 200 µg, with a preferred dose of about 100 µg. A preferred dose volume is about 0.5 ml.

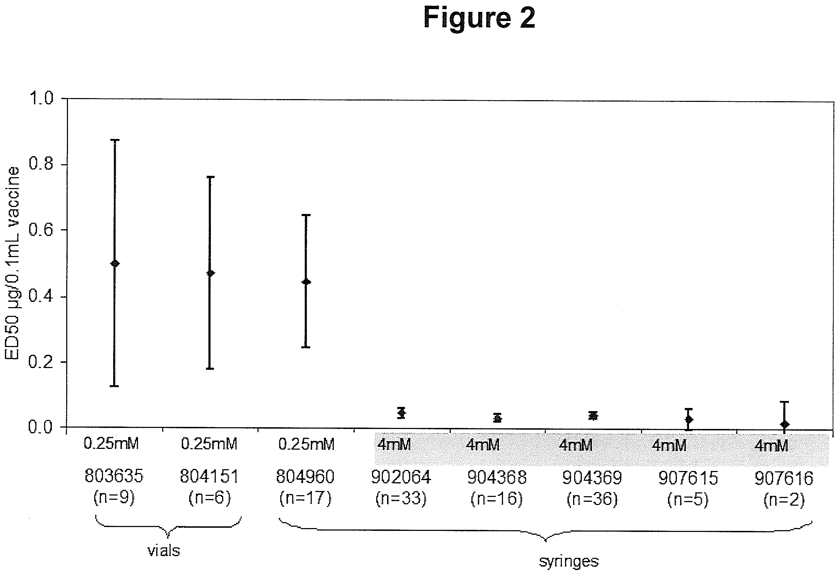

The present disclosure further relates to a vaccine comprising a purified antigen, preferably an anthrax antigen, more preferably anthrax protective antigen (PA) and most preferably a recombinant anthrax protective antigen (rPA), such as that described in figure 2 of WO 2007/122373, pub. 1 November 2007, or that described in WO02/04646, bound to an adjuvant, preferably an alum-based adjuvant. In a preferred embodiment, the present disclosure relates to a sub-unit vaccine comprising alum-bound rPA, at least about 2 mM and most preferably about 4 mM phosphate salt and at about pH 7.1.

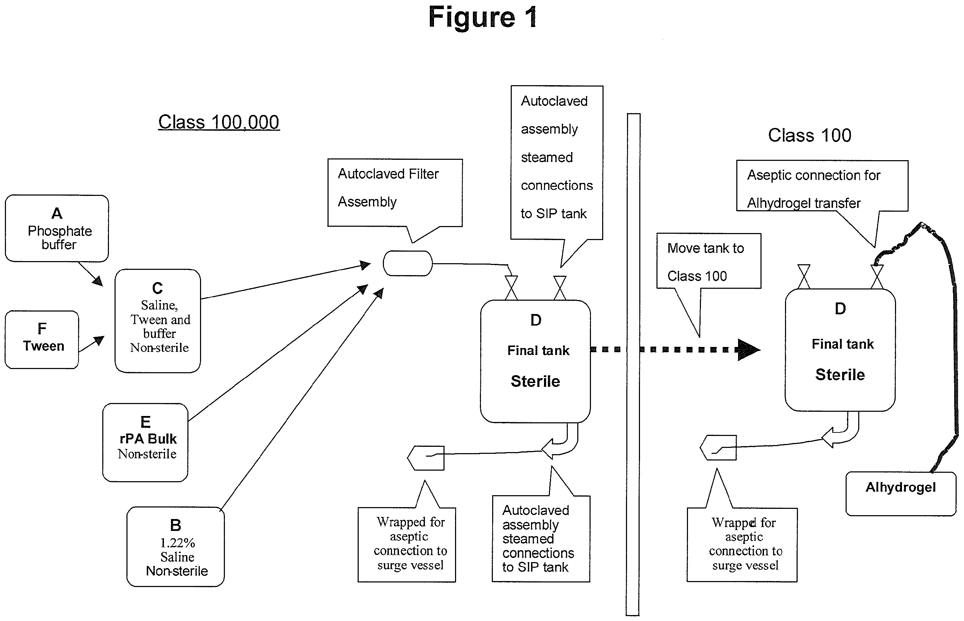

A brief description of the rPA drug product manufacturing process is presented below, along with a process diagram (Figure 1). Drug substance is removed from -70°C storage and allowed to thaw at 2-8°C. While the bulk drug substance solution is thawing, three different solutions are prepared and utilized in the formulation process, namely Solutions A to C. Solution A is the stock 0.5 M phosphate buffer, which is subsequently used to produce Solution C (phosphate buffered 0.9% saline, containing 0.04% Tween 20). It is Solution C that is used to dilute the rPA drug substance to the required concentration. Solution B comprises 1.22% saline, which is used to balance up the osmolality of the alhydrogel. The differences between the original low phosphate process and the modified high phosphate process is the concentration and pH of phosphate in Solution C.

Once the rPA drug substance is thawed and the rPA protein concentration determined by the A280nm in-process assay, this material was sterile filtered along with the appropriate amounts of Solution C and Solution B, into the formulation vessel. Once mixed, sterile alhydrogel was added, with continuous mixing to produce the drug product formulation, i.e. 200ug/ml rPA, 0.26% alhydrogel in phosphate buffered 0.9% saline. The formulation is stirred for 2 hours before being filled into syringes or other appropriate containers.

It was found that for the low phosphate formulation, the pH at release was 5.9 for the resulting materials used in the clinic. In contrast the pH of the high phosphate material was approximately 7.2 at release. Consequently it can be concluded that the increase in phosphate concentration was successful in its aim of controlling the pH at a neutral/physiological value.

A mouse challenge potency assay was used to test and release all batches of drug product. This assay demonstrated that the Drug Products were all potent when manufactured.

In order to be able to make comparative assessments of batch potency, the ED₅₀ value (a measure of effective dose) was derived from the assay data. Surprisingly, this showed that the high phosphate batches produced a significant enhancement in the potency when compared with the low phosphate batches. Moreover, the data from the high phosphate formulation were much more consistent than those from the low phosphate formulation (as indicated by the smaller error bars in Figure 2). Thus, the modification to the phosphate concentration unexpectedly resulted in a marked enhancement of the drug product potency.

To determine the acceptable operating range for phosphate in the rPA drug product process/formulation a series of studies have been performed to evaluate the effect of phosphate concentration upon both the protein and the alum colloid. The results of these studies are presented below.

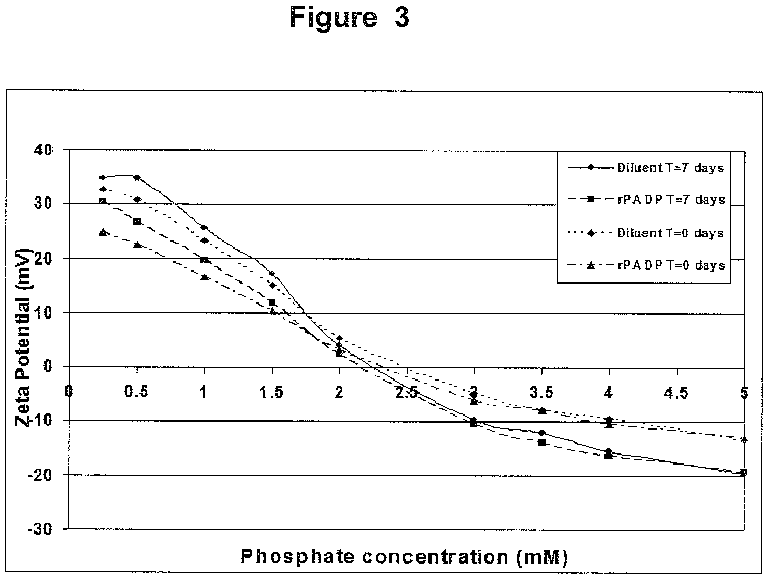

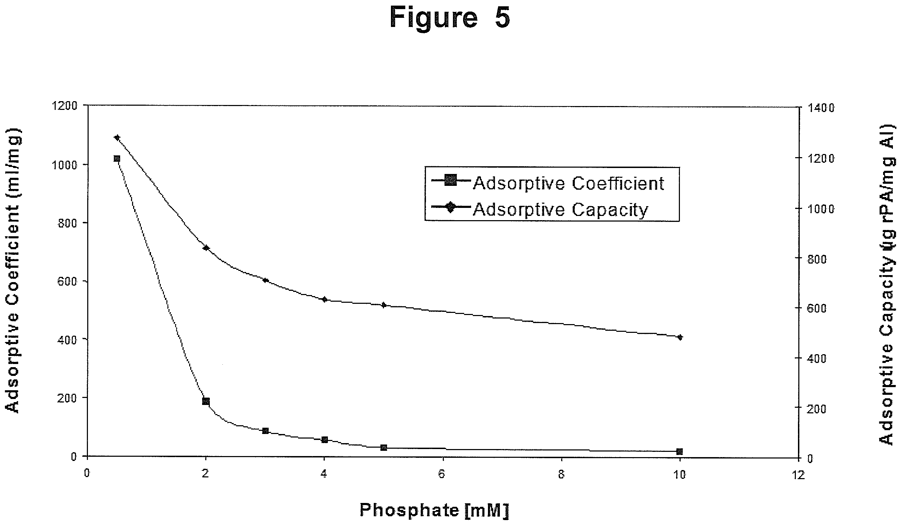

To evaluate the effect of phosphate on the alhydrogel particles the zeta potential of the alum colloidal particles was determined. Drug product and alum diluent were freshly formulated with a phosphate concentration range of 0.25mM to 5mM and the zeta potential was measured at T=day 0 and T=day 7. The results revealed that with increasing phosphate concentration the zeta potential became more negative, with the point of no charge being between 2 and 3mM phosphate (Figure 3). It was also evident that the zeta potential, of both the drug product and the diluent, had not reach equilibrium during formulation but continued to change over the preceding 7 days. This data indicated that the phosphate-alum interaction continued following formulation. However, by extrapolation to the zeta potential drug product batch data, which indicated that the high phosphate drug product batches had a zeta potential of approximately -20 mV (Figure 5), the interaction may be reaching completion by day 7.

Comparison of the rPA drug product and diluent zeta potential values indicated that at low phosphate levels (0.25mM) the bound protein modified the zeta potential of the formulation, however this effect was not evident when the zeta potential became negative with increasing phosphate.

It is well established that phosphate concentration has a marked effect upon the ability of rPA to bind to alhydrogel. Early in drug product development, the effect of phosphate on rPA-alum interaction was evaluated, however this was performed using a phosphate range of 10 to 400mM. This experiment has been repeated, except that this time the effect of ≤10mM was evaluated.

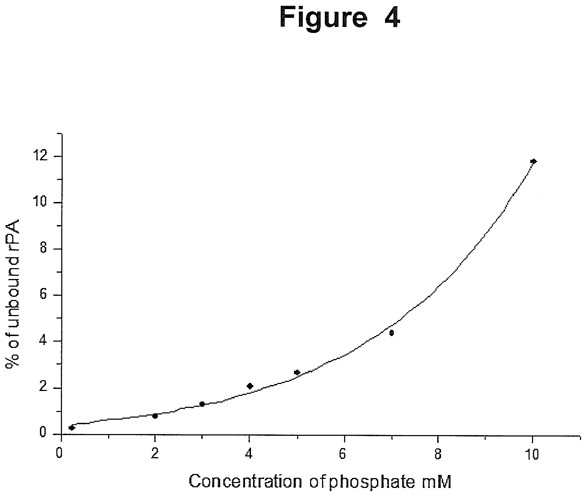

Increasing the concentration of phosphate inhibited rPA-alum binding (Figure 4). At the 10mM level, the % unbound rPA was at 12%, which was within the FDA guideline value of 30% unbound antigen.

Langmuir analysis was used to assess the effect of phosphate on rPA binding to alhydrogel. This analysis is used to determine the adsorptive coefficient (measure of binding strength) and adsorptive capacity (binding capacity) of molecules binding to particles.

As shown in Figure 5, the adsorptive coefficient of rPA binding to alhydrogel was effected by the phosphate concentration, producing a biphasic curve. Following a steep drop in value, the adsorptive coefficient reached a plateau around 3-4 mM phosphate, which suggested that phosphate ions were capable of modulating the strength of rPA-alum binding. Certainly the effect of phosphate ions on the surface charge of the alum, making it more negative, would support this observation. The rPA protein is an acidic protein that would have a stronger interaction with the positively charged alum particles as opposed to the phosphate-modulated negatively charged particles.

What is also evident from the Langmuir analysis is that the adsorptive capacity of the alum also changed with increasing phosphate concentration. This binding capacity parameter was starting to reach a plateau around 3-4mM. It should be noted that this adsorptive capacity was in excess of what was required to bind rPA at 200ug/ml with a concentration of 0.26% alhydrogel.

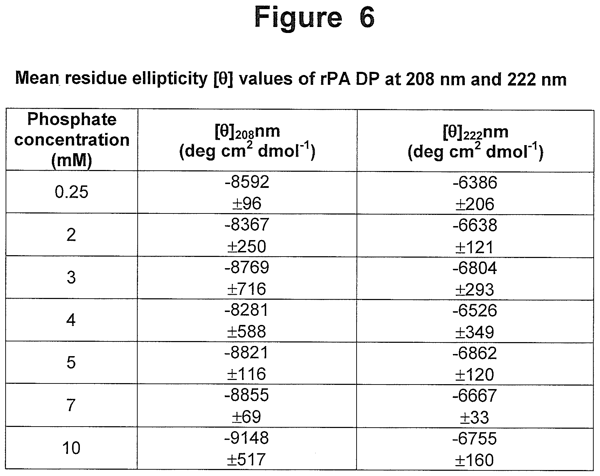

The specialized drug product far UV circular dichroism technique was used to evaluate the effect of phosphate concentration on rPA secondary structure in drug product. rPA Drug Product was formulated with a range of phosphate concentrations (0.25mM to 10mM) and the dichroism spectra were determined between 190nm and 250nm. All the spectra (not shown) produced were characteristic of rPA with a minima at 208nm and a plateau region around 216nm (Figure 6). Furthermore, all the spectra essentially overlapped, indicating that phosphate concentration was not affecting the secondary structure of the rPA protein in freshly formulated drug product.

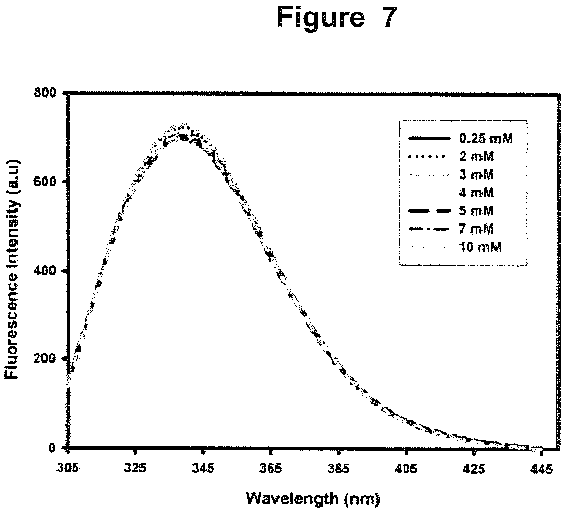

Similarly, intrinsic fluorescence was used to evaluate the effect of phosphate concentration on rPA tertiary structure in drug product. rPA Drug Product was formulated with a range of phosphate concentrations ranging from 0.25mM to 10mM and the fluorescence emission spectra were determined. As shown in Figure 7, all the spectra effectively overlapped each other, irrespective of the phosphate concentration, indicating that there was no effect upon λmax and hence protein tertiary structure

Both intrinsic fluorescence and far UV circular dichroism indicated that phosphate concentration did not effect the rPA secondary/tertiary structure in freshly formulated drug substance. However, because it is known that the rPA protein physically binds to the alum particles and becomes immobilized, we determined whether there was a difference in rPA-alum binding, which did not significantly perturb rPA structure upon binding, a thermal denaturation approach was adopted. DSC analysis was used as an alternative measure of rPA-alum interaction. It was reasoned that immobilizing rPA on alum would reduce heat capacity changes associated with protein melting because of the restricted movement of the anchored protein and this would be detected by DSC.

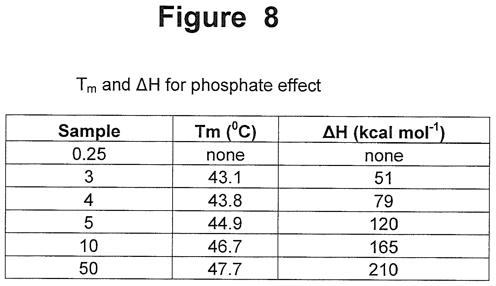

When differential scanning calorimetry (DSC) was applied to freshly formulated drug product over a phosphate concentration range of 0.25 mM to 50 mM, no melting transition was detected with the 0.25 mM formulation. As the phosphate concentration increased to 2 mM, a transition was detected, which increased in both enthalpy and in melting temperature with increased phosphate concentration (Figure 8).

Overall, the DSC supported the observation that increasing phosphate levels reduced the strength of binding of rPA to alum, as evident by the increased heat capacity of the protein. The data also suggests that the stability of the protein increases inversely proportional to the amount it is bound to the alum, as shown be the increasing melting temperature value. Although with the values for 10 and 50 mM phosphate the signal from unbound rPA would start to become significant and would contribute disproportionately to the isotherms.

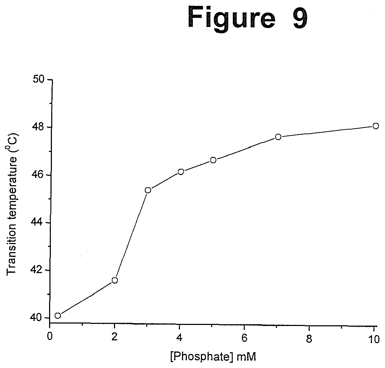

The effectiveness of the thermal denaturation approach to evaluate the rPA structure within drug product, as demonstrated in the DSC data, suggested that this could also be applied to intrinsic fluorescence. Hence, fluorescence intensity/thermal denaturation was used to determine the effect of phosphate on the melting temperatures of the protein bound to alhydrogel (Figure 9). Similar to that shown with DSC, drug products formulated in low phosphate (0.25mM to 2mM) revealed little or no protein melting transition in the 40-50°C range, whereas a transition was detected at 3-10mM, around 46°C. When the data is plotted as melting temperature (midpoint of thermal transition) against phosphate it can be seen that the biphasic curve starts to reach a plateau at 3mM phosphate.

In order to assess whether the increase in phosphate results in the rPA protein binding in a different manner or orientation, studies were performed using the epitope recognition assay (also known as the Drug Product immunoassay). This is effectively a protein structure assay that measures the ability of selected monoclonal antibodies to recognize specific rPA domain 4 epitopes. Domain 4 was chosen since there is evidence that this region of the protein is crucial for conferring antibody protection (Flick-Smith et al, Infect Immun., Vol. 70(3), pp. 1653-6 (2002)), A recombinant carboxy-terminal domain of the protective antigen of Bacillus anthracis protects mice against anthrax infection).

Two monoclonal antibodies raised against separate rPA domain 4 epitopes were used to evaluate rPA binding: (1) a stability-indicating epitope (C3 clone) and (2) a protective/stability-indicating epitope (2D4J clone). Epitope mapping of the two antibodies demonstrated that they were both binding to separate loop regions within rPA domain 4; moreover the 2D4J clone was specifically binding to the receptor binding region, which would account for its protective properties.

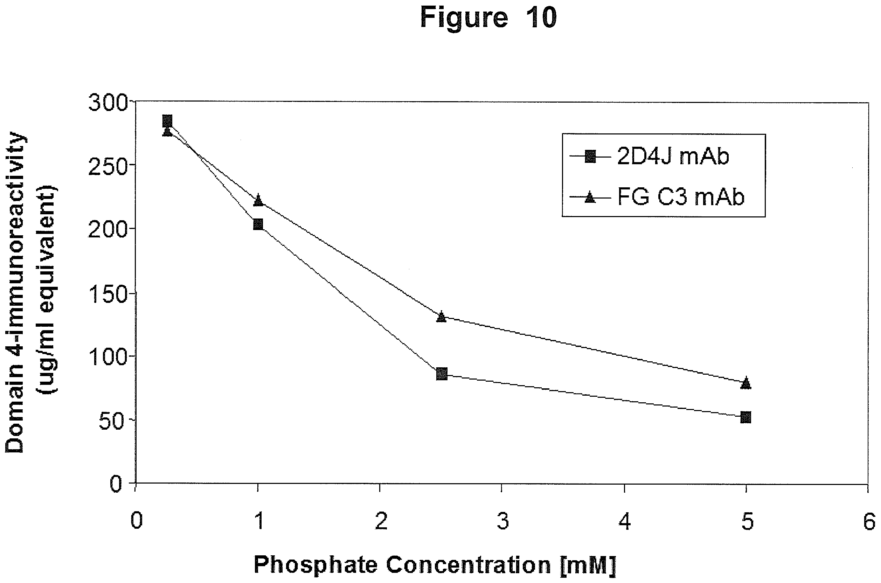

Epitope recognition analysis was performed with increasing concentrations of phosphate. With both monoclonals there was a reduction in antibody binding as a result of the increase in phosphate concentration (Figure 10). With this assay it was not possible to evaluate a large number of phosphate concentrations due to limitations in capacity. However, from the limited number of phosphate concentrations it was seen that the immunoreactivity with both monoclonals declined rapidly up to an inflexion point around 2.5mM, after which the rate of decline in immunoreactivity was decreased. This reduction in immunoreactivity was not due to loss of protein since all rPA effectively bound to the alum.

One unexpected conclusion of the above experiments was that phosphate must henceforth be considered as a drug product formulation critical parameter, due to its effect upon both the formulation characteristics and potency. This is particularly important since up until this point the drug production process was not designed to manufacture rPA drug product with a fixed phosphate concentration. With this old process, rPA drug substance was diluted with a phosphate buffer to reach a specific rPA protein concentration. Since the rPA concentration of the drug substance could potentially vary from the target concentration by a factor of about 17%, the amount of diluting phosphate buffer changed depending on the starting concentration of the drug substance. Since it was the volume of this phosphate that dictated the overall phosphate concentration, the levels of this inorganic ion would vary appropriately. Subsequent modifications to the drug product process have been made to ensure that a constant phosphate concentration was formulated, irrespective of the drug substance concentration at the start of the process.

## Claims

1. A composition for use as an anthrax vaccine, comprising a therapeutically effective amount of an anthrax protective antigen (PA) bound to adjuvant, wherein said adjuvant is alhydrogel (aluminium hydroxide) and containing a phosphate salt at a concentration in the range of 2 to 5 mM.

2. The composition for use as an anthrax vaccine of claim 1, wherein said anthrax protective antigen (PA) is a recombinant anthrax protective antigen (rPA).

3. The composition for use as an anthrax vaccine of claim 2, wherein said phosphate salt concentration is present in the range of 3 to 5 mM.

4. The composition for use as an anthrax vaccine of claim 2, wherein said phosphate salt concentration is present in the range of 3.5 to 4.5 mM.

5. The composition for use as an anthrax vaccine of claim 2, wherein said phosphate salt concentration is present in the range of 3.8 to 4.2 mM.

6. The composition for use as an anthrax vaccine of claim 2, wherein said phosphate salt concentration is 4.0 mM.

7. The composition for use as an anthrax vaccine of claim 1, wherein the pH of said vaccine is 7.0 to 7.2.

8. The composition for use as an anthrax vaccine of claim 1, wherein said alhydrogel (aluminium hydroxide) is present at 0.15 to 0.35%.

9. The composition for use as an anthrax vaccine of claim 1, wherein said alhydrogel (aluminium hydroxide) is present at 0.20 to 0.30%.

10. The composition for use as an anthrax vaccine of claim 2, wherein said rPA is in the range of 10 to 300 µg/ml.

11. The composition for use as an anthrax vaccine of claim 2, wherein said rPA is in the range of 50 to 300 µg/ml.

12. The composition for use as an anthrax vaccine of claim 2, wherein said rPA is in the range 100 to 300 µg/ml.

13. The composition for use as an anthrax vaccine of claim 2, wherein said rPA is in the range 150 to 250 µg/ml.

14. The composition for use as an anthrax vaccine of claim 2, wherein said rPA is present at 200 µg/ml, said phosphate is present at 4mM, said alhydrogel is present at 0.26% by weight and wherein the pH of said vaccine is 7.1.

15. The composition for use as an anthrax vaccine of claim 2, wherein said rPA is present at 200 µg/ml.

16. The composition for use as an anthrax vaccine of claim 2, wherein said phosphate salt concentration is present in the range of 2.5 to 4.5 mM

## Patentansprüche

1. Zusammensetzung zur Verwendung als Anthrax-Impfstoff, umfassend eine therapeutisch wirksame Menge eines Anthrax-protektiven Antigens (PA), das an ein Adjuvans gebunden ist, wobei das Adjuvans Alhydrogel (Aluminiumhydroxid) ist, und enthaltend ein Phosphatsalz in einer Konzentration im Bereich von 2 bis 5 mM.

2. Zusammensetzung zur Verwendung als Anthrax-Impfstoff nach Anspruch 1, wobei das Anthrax-protektive Antigen (PA) ein rekombinantes Anthrax-protektives Antigen (rPA) ist.

3. Zusammensetzung zur Verwendung als Anthrax-Impfstoff nach Anspruch 2, wobei die Phosphatsalz-Konzentration im Bereich von 3 bis 5 mM vorliegt.

4. Zusammensetzung zur Verwendung als Anthrax-Impfstoff nach Anspruch 2, wobei die Phosphatsalz-Konzentration im Bereich von 3,5 bis 4,5 mM vorliegt.

5. Zusammensetzung zur Verwendung als Anthrax-Impfstoff nach Anspruch 2, wobei die Phosphatsalz-Konzentration im Bereich von 3,8 bis 4,2 mM vorliegt.

6. Zusammensetzung zur Verwendung als Anthrax-Impfstoff nach Anspruch 2, wobei die Phosphatsalz-Konzentration 4,0 mM beträgt.

7. Zusammensetzung zur Verwendung als Anthrax-Impfstoff nach Anspruch 1, wobei der pH-Wert des Impfstoffs 7,0 bis 7,2 beträgt.

8. Zusammensetzung zur Verwendung als Anthrax-Impfstoff nach Anspruch 1, wobei das Alhydrogel (Aluminiumhydroxid) mit 0,15 bis 0,35% vorliegt.

9. Zusammensetzung zur Verwendung als Anthrax-Impfstoff nach Anspruch 1, wobei das Alhydrogel (Aluminiumhydroxid) mit 0,20 bis 0,30% vorliegt.

10. Zusammensetzung zur Verwendung als Anthrax-Impfstoff nach Anspruch 2, wobei das rPA im Bereich von 10 bis 300 µg/ml ist.

11. Zusammensetzung zur Verwendung als Anthrax-Impfstoff nach Anspruch 2, wobei das rPA im Bereich von 50 bis 300 µg/ml ist.

12. Zusammensetzung zur Verwendung als Anthrax-Impfstoff nach Anspruch 2, wobei das rPA im Bereich 100 bis 300 µg/ml ist.

13. Zusammensetzung zur Verwendung als Anthrax-Impfstoff nach Anspruch 2, wobei das rPA im Bereich 150 bis 250 µg/ml ist.

14. Zusammensetzung zur Verwendung als Anthrax-Impfstoff nach Anspruch 2, wobei das rPA mit 200 µg/ml vorliegt, das Phosphat mit 4 mM vorliegt, das Alhydrogel mit 0,26 Gew.-% vorliegt und wobei der pH-Wert des Impfstoffs 7,1 beträgt.

15. Zusammensetzung zur Verwendung als Anthrax-Impfstoff nach Anspruch 2, wobei das rPA mit 200 µg/ml vorliegt.

16. Zusammensetzung zur Verwendung als Anthrax-Impfstoff nach Anspruch 2, wobei die Phosphatsalz-Konzentration im Bereich von 2,5 bis 4,5 mM vorliegt.

## Revendications

1. Composition destinée à être utilisée comme vaccin contre le charbon, comprenant une quantité thérapeutiquement efficace d'un antigène protecteur contre le charbon (PA) fixé à un adjuvant, où ledit adjuvant est de alhydrogel (hydroxyde d'aluminium) et contenant un sel de phosphate à une concentration comprise dans la fourchette de 2 à 5 mM.

2. Composition destinée à être utilisée comme vaccin contre le charbon selon la revendication 1, où ledit antigène protecteur contre le charbon (PA) est un antigène protecteur contre le charbon recombinant (rPA).

3. Composition destinée à être utilisée comme vaccin contre le charbon selon la revendication 2, où ladite concentration du sel de phosphate se situe dans la fourchette de 3 à 5 mM.

4. Composition destinée à être utilisée comme vaccin contre le charbon selon la revendication 2, où ladite concentration du sel de phosphate se situe dans la fourchette de 3,5 à 4,5 mM.

5. Composition destinée à être utilisée comme vaccin contre le charbon selon la revendication 2, où ladite concentration du sel de phosphate se situe dans la fourchette de 3,8 à 4,2 mM.

6. Composition destinée à être utilisée comme vaccin contre le charbon selon la revendication 2, où ladite concentration du sel de phosphate est de 4,0 mM.

7. Composition destinée à être utilisée comme vaccin contre le charbon selon la revendication 1, où le pH dudit vaccin est de 7,0 à 7,2.

8. Composition destinée à être utilisée comme vaccin contre le charbon selon la revendication 1, où ledit alhydrogel (hydroxyde d'aluminium) est présent à raison de 0,15 à 0,35%.

9. Composition destinée à être utilisée comme vaccin contre le charbon selon la revendication 1, où ledit alhydrogel (hydroxyde d'aluminium) est présent à raison de 0,20 à 0,30%.

10. Composition destinée à être utilisée comme vaccin contre le charbon selon la revendication 2, où ledit rPA est dans la fourchette de 10 à 300 µg/ml.

11. Composition destinée à être utilisée comme vaccin contre le charbon selon la revendication 2, où ledit rPA est dans la fourchette de 50 à 300 µg/ml.

12. Composition destinée à être utilisée comme vaccin contre le charbon selon la revendication 2, où ledit rPA est dans la fourchette de 100 à 300 µg/ml.

13. Composition destinée à être utilisée comme vaccin contre le charbon selon la revendication 2, où ledit rPA est dans la fourchette de 150 à 250 µg/ml.

14. Composition destinée à être utilisée comme vaccin contre le charbon selon la revendication 2, où ledit rPA est présent à 200 µg/ml, ledit phosphate est présent à 4 mM, ledit alhydrogel est présent à 0,26% en poids et où le pH dudit vaccin est de 7,1.

15. Composition destinée à être utilisée comme vaccin contre le charbon selon la revendication 2, où ledit rPA est présent à 200 µg/ml.

16. Composition destinée à être utilisée comme vaccin contre le charbon selon la revendication 2, où ladite concentration du sel de phosphate se situe dans la fourchette de 2,5 à 4,5 mM.
